(19) Europäisches Patentamt
European Patent Office
Office européen des brevets

(11) EP 2 633 906 A1

(12) **EUROPEAN PATENT APPLICATION**
published in accordance with Art. 153(4) EPC

(43) Date of publication:
**04.09.2013 Bulletin 2013/36**

(21) Application number: **11836359.7**

(22) Date of filing: **26.10.2011**

(51) Int Cl.:
***B01J 23/52*** (2006.01)
***C07B 61/00*** (2006.01)
***C07C 211/07*** (2006.01)
***H01M 4/90*** (2006.01)
***C07C 209/48*** (2006.01)

(86) International application number:
**PCT/JP2011/074715**

(87) International publication number:
**WO 2012/057226 (03.05.2012 Gazette 2012/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2010 JP 2010240341**

(71) Applicant: **N.E. Chemcat Corporation**
**Tokyo 105-6124 (JP)**

(72) Inventors:
- **MIZUSAKI, Tomoteru**
  **Bando-shi**
  **Ibaraki 306-0608 (JP)**
- **NAGAMORI, Kiyotaka**
  **Bando-shi**
  **Ibaraki 306-0608 (JP)**

(74) Representative: **Tönhardt, Marion**
**Boehmert & Boehmert**
**Pettenkoferstrasse 20-22**
**80336 München (DE)**

# (54) REDUCTION CATALYST COMPRISING PALLADIUM-GOLD ALLOY

(57) Provide is a reduction catalyst, which comprises conductive carbon and a palladium-gold alloy supported on the carbon, wherein the alloying degree of said alloy is 50 to 100%. This palladium-supported catalyst has an excellent reduction ability, and exhibits a high conversion ratio and preferably an excellent selectivity when used for hydrogenation reaction. The reduction catalyst is useful as a catalyst for oxygen reduction or as a catalyst for hydrogenation. The catalyst for oxygen reduction is useful as a cathode electrode catalyst for polymer electrolyte fuel cells. The cathode electrode catalyst can be used for a cathode electrode for polymer electrolyte fuel cells.

EP 2 633 906 A1

## Description

Technical Field

The present invention relates to a reduction catalyst containing palladium-gold alloy.

Background Art

The palladium-supported catalysts in which palladium or palladium alloy with the other metals is supported on a supporting material such as alumina, carbon, etc. have been put to practical use as a heterogenious catalyst in a wide range of application such as hydrogenation of olefins, acetylene, nitro groups, ketones, aldehydes, nitriles, etc., oxidation of hydrogen, hydrocarbons, carbon monoxide, etc., and oxidative acetoxylation reaction of olefins.

As such palladium supported catalysts have been disclosed, for example, Pd-C catalysts and Pd-$Al_2O_3$ catalysts, and these catalysts can be used for hydrogenation reactions under elevated temperature and increased pressure (Non-Patent Document 1).

Prior Art Document

Patent Document

Non-Patent Document 1: Shigeo Nishimura,Yuzuru Takagi, "Catalytic Hydrogenation Reaction, Application in Organic Synthesis", April 10, 1987, published by Tokyo Kagaku Dojin Co. Ltd., p. 34-37

Summary of Invention

Problems to be solved by Invention

Conventional palladium-supported catalysts are low in reducibility and are insufficient in selectivity in some reactions. Thus, an object of the present invention is to provide a palladium-supported catalyst which is excellent in reducibility and exhibits a high conversion ratio and preferably an excellent selectivity when used in a hydrogenation reaction.

Means for Solution of the Problems

As the results of intensive researches, the present inventors have noted an alloying degree of an alloy as an index for evaluating the microstructure of the alloy, and have completed the present invention.

Namely, the present invention provides reduction catalyst which comprises conductive carbon and palladium-gold alloy supported on the said carbon, wherein an alloying degree of the said alloy is in a range of from 50 to 100%.

The reduction catalyst according to the invention is an oxygen-reduction catalyst in one embodiment, and is a hydrogenation catalyst in other embodiment. The oxygen-reduction catalyst is a cathode catalyst for polymer electrolyte fuel cells in one embodiment.

Further, the present invention provides a cathode catalyst for polymer electrolyte fuel cells, wherein the cathode catalyst is used.

Further, the present invention provides a method for reducing oxygen which comprises contacting the reduction catalyst according to the present invention with oxygen.

Further, the present invention provides a method for hydrogenating an organic compound having reducible functional groups, which comprises contacting the reduction catalyst according to the present invention with the organic compound having the reducible functional groups. Examples of the reducible functional groups include, for example, carbon-carbon double bond-containing groups (such as alkenyl group), carbon-carbon triple bond-containing groups (such as alkynyl group), nitro group, carbonyl group and cyano group. Examples of the organic compounds include, for example, olefins, acetylene, nitro compounds, ketones, aldehydes, and nitriles.

Further, the present invention provides use of the reduction catalyst according to the present invention as an oxygen-reduction catalyst.

Further, the present invention provides use of the reduction catalyst according to the present invention as a hydrogenation catalyst.

Effects of the Invention

The reduction catalyst according to the present invention is excellent in reducibility, and thus is useful as, for example, an oxygen-reduction catalyst, and further as a cathode catalyst for polymer electrolyte fuel cell. A cathode according to the invention in which the cathode catalyst is used is useful for polymer electrolyte fuel cell which are expected to be used as stationary power source unit for household use and mobile power supply system for automobiles. The reduction catalyst according to the present invention is also useful as a hydrogenation catalyst which exhibits a high conversion ratio and moreover an excellent selectivity in, for example, a reaction for hydrogenating an aliphatic nitrile compound to convert it into a primary amine.

Brief Description of Drawings

FIG. 1 shows an approximation graph indicating a theoretical value of each of lattice spacing (face-to-face distances) *d* of alloy crystals having various Pd contents (mol %).
FIG. 2 shows an XRD pattern for a Pd-Au alloy having Pd content of 90 mol %, obtained by using X ray (Cu-K$\alpha$) having a wavelength of 1.540598 angstrom.
FIG. 3 shows another XRD pattern for a Pd-Au alloy having Pd content of 90 mol %, obtained by using X ray (Cu-K$\alpha$) having a wavelength of 1.540598 angstrom.
FIG. 4 is a graph showing current-potential curves of catalysts at a rotation number of 1600 rpm. In the drawing, (A)-(D) correspond to samples A-D, respectively, and (E) corresponds to a palladium-supported carbon catalyst.
FIG. 5 is a graph showing a relation between alloying degree and oxygen-reduction current.

Detailed description of the Invention

The invention will be explained below in more detail.

(1) Reduction catalyst

1-1. Formation of palladium-gold alloy-supported carbon catalyst

Suitable conductive carbon support materials include, for example, acetylene black- or furnace black-based carbon powder. The mol ratio of palladium to gold in the supported palladium-gold alloy is preferably from 9.5: 0.5 to 7 : 3.
The palladium-gold alloy-supported carbon can be formed by the following procedures;

- Conductive carbon powder is uniformly dispersed in pure water.
- Mixed solution of palladium salt and gold salt is used to produce the alloy particles to be supported on carbon powder. The palladium salt such as, but not limited to, sodium chloropalladate, palladium chloride, palladium nitrate or the like can be used, and the gold salt such as, but not limited to, chloroauric acid, sodium chloroaurate or the like can be used. They are weighed so that the total weight of the alloy to be produced comes to preferably 10 to 40% by weight, more preferably 20 to 30% by weight of the overall weight of the catalyst.
- Palladium ion and gold ion are adsorbed on the conductive carbon support by a chemical adsorption method.
- Then, the metal ions adsorbed on the conductive carbon support are chemically reduced. As a reducing agent, can be used hydrazine, formaldehyde, sodium formate, formic acid, etc.

Through the above procedures, particles consisting of a palladium-gold alloy can be formed on the conductive carbon support.

1-2. Alloying degree

- In the reduction catalyst according to the invention, an alloying degree of a palladium-gold alloy is usually 50 to 100%, preferably 70 to 100%, more preferably 80 to 100%, further more preferably 90 to 100%. If the alloying degree is less than the lower limit, the resulting catalyst may have poor reducibility in some cases.

In the present description, the alloying degree of a palladium-gold alloy refers to an index determined as

described below from the X-ray diffraction (XRD) data, specifically the measured values of the peak top position as a diffraction angle $2\theta$ of the (220) plane and accordingly calculated value of lattice spacing $d$. In other word, it is possible to determine the crystal structure of a palladium-gold alloy catalyst from the X-ray diffraction pattern by calculating the lattice spacing $d$ from the characteristic diffraction angle $2\theta$, wherein the deviations of the lattice spacing $d$ of the empirically prepared palladium-gold alloy from those of pure palladium and pure gold are calculated. Other deviation values are calculated by using the lattice spacing $d$ of the ideal palladium-gold alloy, pure palladium and pure gold in the same manner. Finally, the ratios of those deviations between the empirical and the ideal alloys are allocated proportionately to the mol fractions of palladium and gold present in the catalyst according to Vegard's Law; and the resulting value is used as an index of the alloying degree of the whole catalyst.

First, data used as standard are shown in Table 1. Those data were obtained from XRD patterns of pure palladium (Pd content: 100 mol %) and pure gold (Pd content: 0 mol %) respectively at wavelength of 1.540598 angstrom (Cu-K$\alpha$) by using Bragg's condition:

$$2d \sin \theta = n\lambda$$

wherein, n=1, $\lambda$=1.540598 angstrom

[Table 1]

| | Pd(220) | Au(220) |
|---|---|---|
| $d$ (angstrom) | 1.3754 | 1.4420 |
| $2\theta$(deg) | 68.119 | 64.578 |
| Pd content (mol %) | 100 | 0 |

An approximation graph indicating the theoretical values of lattice spacing d of each alloy were drawn in the range of Pd content between 0 to 100% based on the assumption that the lattice spacing of alloy changes linearly depending on Pd content in mol % (Fig. 1). Using that approximation graph, the theoretical values of $d$ and $2\theta$ of alloys with various Pd content were calculated from the above-mentioned Bragg's condition, as shown in Table 2.

[Table 2]

| | Pd content (mol %) | | | | | |
|---|---|---|---|---|---|---|
| | 100 | 90 | 80 | 70 | 50 | 0 |
| $d$ (angstrom) | 1.3754 | 1.3821 | 1.3887 | 1.3954 | 1.4087 | 1.4420 |
| $2\theta$(deg) | 68.119 | 67.746 | 67.378 | 67.014 | 66.298 | 64.578 |

Based on the above results, the alloying degree was defined from the theoretical and the observed value of $d$ as follows. Namely, the alloying degree of each component metal is calculated by the following equation, in which the alloying degree of palladium-gold alloy is defined as an allocation of the alloying degree in proportion to the mol fraction of each component metal.

$$\begin{aligned}&\text{Alloying degree (\%)}\\&= \Sigma(\text{mol fraction of component metal})_i \cdot (\text{alloying degree of component metal})_i\\&= [\text{Pd mol fraction}] \cdot [\text{alloying degree of Pd}] + [\text{Au mol fraction}] \cdot [\text{alloying degree of Au}]\end{aligned}$$

In the above equation, Pd mol fraction is calculated from the equation: (Pd content) /100, and Au mol fraction was calculated from the equation: 1 - (Pd mol fraction).

Meanwhile, the alloying degree of component metal in the above equation is calculated from the following equation:

$$\text{Alloying degree (\%) of component metal} = (|d\text{ (measured)} - d\text{(standard data)}| / \{d\text{ (theoretical)} - d\text{(standard data)}\} \times 100$$

In the above equation, the *d* (standard data) means the value *d* shown in Table 1. Namely, when the component metal is palladium, *d* (standard data) = 1.3754 angstrom, and when the component metal is gold, d (standard data) = 1.4420 angstrom.

Meanwhile, *d* (theoretical) in the above equation is the value calculated from the approximation curve shown in Table 1. For example, when Pd content is 90 mol %, *d* (theoretical) = 1.3821 angstrom (see Table 2).

Further, in the above equation, *d* (measured) is the value calculated from Bragg's condition based on the results of XRD measurement. In the case where the peak of the (220) plane is observed as a single peak in the XRD, the value is calculated from the position of such single peak. On the contrary, in the case where the peaks of the (220) plane is observed as split two peaks in the XRD, the value is calculated by allocating the peak at higher angle to palladium and the peak at lower angle to gold.

For example, in the case where a palladium-gold alloy with Pd content of 90 mol % provides an XRD pattern shown in Fig. 2 using an X ray (Cu-K$\alpha$) with wavelength of 1.540598 angstrom, where the peak of (220) plane is observed as a single peak, these values that $2\theta$(measured) = 67.759 degrees and *d* (measured) = 1.3818 angstrom are calculated from Bragg's condition, as an identical number for palladium and gold. Accordingly, the alloying degree of each component metal is calculated as shown in Table 3, and the alloying degree of the palladium-gold alloy is calculated to be 96.02%.

[Table 3]

| | Mol Fraction | $2\theta$ (measured) (°) | $2\theta$ (calculated) (°) | *d* (measured) (angstrom) | *d* (theoretical) (angstrom) | *d* (standard data) (angstrom) | Alloying degree of component metal (%) |
|---|---|---|---|---|---|---|---|
| Pd | 0.9 | 67.759 | 67.746 | 1.3818 | 1.3821 | 1.3754 | 95.52 |
| Au | 0.1 | 67.759 | 67.746 | 1.3818 | 1.3821 | 1.4420 | 100.50 |

$$\begin{aligned}\text{Alloying degree (\%) of palladium-gold alloy} &= \\ &= [\text{Pd mol fraction}] \cdot [\text{alloying degree of Pd}] + [\text{Au mol fraction}] \cdot [\text{alloying degree of Au}] \\ &= 0.9 \times 95.52 + 0.1 \times 100.50 \\ &= 96.02.\end{aligned}$$

In the different case where a palladium-gold alloy with Pd content of 90 mol % provides an XRD pattern shown in Fig. 3 using an X ray (Cu-K$\alpha$) with wavelength of 1.540598 angstrom, where the peak of (220) plane is observed as two split peaks, the peak at $2\theta$(measured) of 68.162 degree is allocated to palladium and the other peak at $2\theta$(measured) of 65.215 degree is allocated to gold. Accordingly, from Bragg's condition, *d* (measured) is calculated as 1.3746 angstrom for palladium and 1.4294 angstrom for gold, respectively. Then, an alloying degree of each component metal is calculated to be the values in Table 4, and the alloying degree of the palladium-gold alloy is calculated to be 12.85%.

[Table 4]

| | Mol fraction | $2\theta$ (measured) (°) | $2\theta$ (theoretical) (°) | $d$(measured) (angstrom) | $d$(theoretical) (angstrom) | $d$ (standard data) (angstrom) | Alloying degree of component metal (%) |
|---|---|---|---|---|---|---|---|
| Pd | 0.9 | 68.162 | 67.746 | 1.3746 | 1.3821 | 1.3754 | 11.94 |
| Au | 0.1 | 65.215 | 67.746 | 1.4294 | 1.3821 | 1.4420 | 21.04 |

$$\text{Alloying degree (\%) of palladium-gold alloy} = 0.9 \times 11.94 + 0.1 \times 21.04 = 12.85.$$

The alloying degree can be controlled as follows. Namely, in the "1-1. Formation of palladium-gold alloy-supported carbon" in the above, an alloying degree can be controlled by adjusting pH during addition of the reducing agent. The pH is preferably in a range from 9.0 to 13.0, more preferably from 10.0 to 13.0, further more preferably from 11.0 to 13.0. If the pH is within the above-mentioned range, the alloying degree of the resulting palladium-gold alloy can be readily controlled to a value within a range from 50 to 100%. For adjusting the pH, conventional pH adjusting chemicals can be used without particular limitation. Examples of the pH adjusting chemical include, for example, potassium hydroxide, sodium hydroxide, potassium carbonate and sodium carbonate.

1-3. Use of catalyst

The reduction reactions for which the catalyst of this invention can be used include, but not limited to, for example, oxygen-reduction reactions; hydrogenation reactions of olefins, acetylene, nitro groups, ketones, aldehydes or nitriles; hydrogenation decomposition reactions of benzyl ethers, benzyl esters, or benzyl oxycarbonyl groups.

• Oxygen-reduction catalyst

In the case where the catalyst of this invention is an oxygen-reduction catalyst used for an oxygen-reduction reaction, examples thereof include cathode catalyst for polymer electrolyte fuel cell.

• Hydrogenation catalyst

In the case where the catalyst of the invention is a hydrogenation catalyst used for a hydrogenation reaction, examples of the hydrogenation include, but not limited to, the reaction in which hydrogen is added to aliphatic nitrile compound to convert it to primary amine. Examples of the aliphatic nitrile compound include an aliphatic nitrile compound represented by a general formula: $R^1$-CN, and examples of the primary amine include a primary amine represented by a general formula: $R^1$-$CH_2$-$NH_2$. In these formulas, $R^1$ denotes an unsubstituted or substituted alkyl group. Examples of $R^1$ include alkyl groups of 1 to 20 carbon atoms, preferably 3 to 10 carbon atoms, such as methyl, ethyl, propyl, isopropyl, butyl, t-butyl, pentyl, hexyl and octyl groups; and substituted alkyl groups in which a part or all of the hydrogen atoms in such alkyl groups are substituted with halogen atoms such as fluorine atoms, bromine atoms or chlorine atoms, hydroxyl group, amino group, carbonyl group, oxo group (i.e. =O), alkoxy group or thiol group, such as chloromethyl group, bromoethyl group and 3,3,3-trifluoropropyl group.

The catalytic hydrogenation reaction to convert a nitrile to an amine proceeds in the presence of various metal catalysts (Co, Ni, Rh, Pd, Pt, etc.). However, it is known that secondary amines and tertiary amines easily generate in addition to generation of primary amines as shown by the chemical formula shown below. The ratio of these amines varies depending on reaction conditions such as catalyst type, temperature, pressure, solvent and the like. Therefore, the selection of catalyst type and reaction conditions is important to obtain primary amines as the main product. For example, in a reaction at higher temperature, condensation reaction accompanied by elimination of ammonia is promoted, and thus secondary amines and tertiary amines are predominantly produced. On the other hand, in the presence of excess ammonia, an equilibrium of the step of the reaction between imine intermediate and primary amine to produce secondary amine is suppressed, whereby resulting in decrease of production of secondary amines and tertiary amines. In other example, by conducting the reaction in acid solvent or acylation solvent such as acetic anhydride, primary amines can be predominantly produced. It is believed that such result comes from the formation of ammonium salt from the acid and the primary amine and thus addition of the primary amine to imine intermediates is suppressed.

[Chemical formula 1]

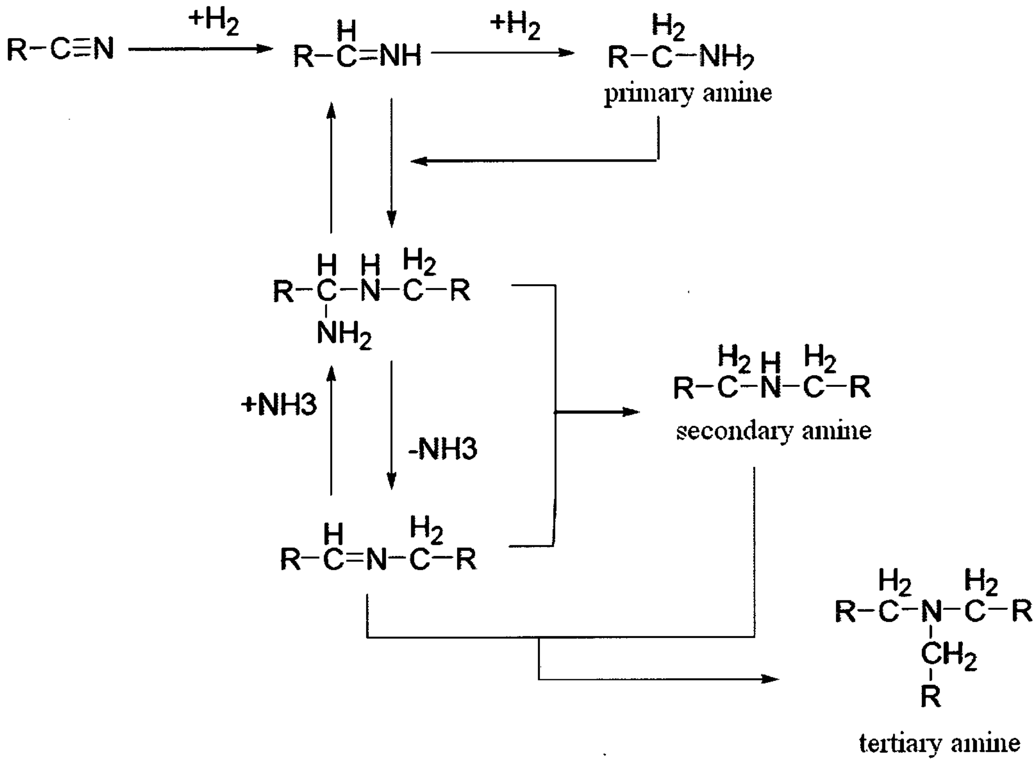


The hydrogenation catalyst according to the present invention can remarkably enhance selectivity of primary amine synthesis in reaction for converting aliphatic nitrile compounds to amines by addition of hydrogen.

(2) Cathode for polymer electrolyte fuel cells

Catalyst layer is formed on gas diffusion layer to prepare an anode and a cathode, and then an ion-exchange membrane is placed between the anode and the cathode with the catalyst layer inside, which is pressed to form membrane-electrode assembly (MEA). The both electrodes are formed by the following preparation method.

For preparing the gas diffusion layer, conductive carbon is blended with a dispersing agent, an aqueous solution of Teflon (registered trade mark) is added thereto for retention of water- repellency, the resulting mixture is applied to conductive carbon sheet, and the resulting product is dried.

Thereafter, the product is molded by a hot-pressing machine to a sheet-shaped product.

For preparing the catalyst layer, catalyst is blended with pure water, Nafion solution or the like is added thereto according to a conventional method, and after further blending, the resulting mixture is coated on the gas diffusion layer.

Then, after drying, the catalyst layer is formed on the gas diffusion layer.

An electrode sheet is thus prepared.

Examples

The present invention will be explained below by way of examples and comparative example which are not intended to restrict the invention.

(1) Preparation of a palladium-gold alloy-supported carbon catalyst

Using 2.5 L of pure water, 44 g of acetylene black used as catalyst support was dispersed therein, and the resulting dispersion was heated to 95 °C to give a uniform suspension of the catalyst support. Separately, 500 mL of pure water was prepared, and sodium chloropalladate in an amount of 15.8 g as palladium metal basis and chloroauric acid in an amount of 3.3 g on as gold metal basis were dissolved in the water to give a mixed solution of palladium salt and gold salt. Then the mixed solution of palladium salt and gold salt was added dropwise to the suspension of the

catalyst support to give a renewed suspension. The renewed suspension was cooled to room temperature. Then formaldehyde and potassium hydroxide were dissolved in 500 mL of pure water to give a reducing solution, and the reducing solution was added dropwise over 60 minutes to the suspension which had been cooled to room temperature. After dropwise addition, the resulting mixture was heated to conduct reduction.
Thus, particles of palladium and gold were supported on the surface of the acetylene black as the support material.
After cooling the reaction liquid, solid matter was filtered off and rinsed.
Thereafter, drying was effected at about 70 °C in the atmosphere, and the solid matter was pulverized to give a catalyst in which the palladium-gold alloy was supported on the acetylene black used as a support.
Incidentally, samples A to D were prepared by adjusting the pH of the reducing solution to 13.0, 11.0, 10.0 and 8.0, respectively.

In the present preparation of the catalyst, the theoretical mol ratio of palladium to gold was 9:1, and the contents of the supported elements relative to the weight of the catalyst were 25 % by weight of palladium and 5.2% by weight of gold according to ICP analysis.
Incidentally, catalysts having a theoretical mol ratio of palladium to gold of 8:2 and 7:3 were also prepared.

(2) Determination of alloying degree

XRD measurement was conducted on the samples A to D obtained in the procedure (1) above to give X-ray diffraction patterns. Upon observation of the peaks of the (220) plane in these XRD patterns, it was shown that samples A to C have a single peak while sample D has two peaks. In sample D, the peak at higher angle was dealt as corresponding to palladium and the peak at lower angle was dealt as corresponding to gold. From the peak position of the (220) plane, an alloying degree was calculated. The results are shown in Table 5.

[Table 5]

| Sample | Pd content (mol %) | Au content (mol %) | 2θ (measured) (°) | 2θ (theoretical) (°) | d (measured) (angstrom) | d (theoretical) (angstrom) | Alloying degree (%) |
|---|---|---|---|---|---|---|---|
| A | 90 | 10 | 67.758 | 67.746 | 1.3818 | 1.3821 | 96.02 |
| B | 90 | 10 | 67.804 | 67.746 | 1.3810 | 1.3821 | 85.41 |
| C | 90 | 10 | 67.893 | 67.746 | 1.3794 | 1.3821 | 64.18 |
| D | 90 | 10 | 68.162 | 67.746 | 1.3746 | 1.3821 | 12.85 |
| | | | 65.215 | 67.746 | 1.4294 | 1.3821 | |

(3) Evaluation of oxygen reducibility

In order to evaluate the oxygen reducibility of palladium-gold alloy-supported carbon catalysts, the rotating disk electrode measurement of the samples A-D was conducted. As the evaluation apparatus was used electrochemical measurement system (HZ-3000) (Hokuto Denko Corporation). The apparatus is composed of rotating electrodes, control units and electrolysis cells all manufactured by Hokuto Denko Corporation. Preparation of the electrode was conducted by applying catalyst to the glassy carbon disk electrode attached to the rotating electrode according to the following procedures. Incidentally, as a reference catalyst, 30wt.% palladium-supported carbon catalyst (referred to as sample E) was used.

1. A solution containing 10 mg of a catalyst, 5 mL of pure water and a 5 wt.% Nafion solution (supplied by Aldrich Corporation) was preliminarily prepared, and subjected to ultrasonic dispersion for 15 minutes.
2. 10 μL of the solution was dropped on the glassy carbon.
3. The resulting glassy carbon was dried overnight at room temperature.

Evaluation conditions are as follows:

1. 0.1 M perchloric acid was used as an electrolyte, a silver/silver chloride electrode was used as a reference electrode, and a platinum mesh with platinum black was used as a counter electrode.

2. After degassing the electrolyte with argon gas for 30 minutes, the rotating electrode was immersed therein and was subjected to a pre-treatment at a scanning potential of 85mV to 1085 mV vs. reversible hydrogen electrode (RHE) and a scanning speed of 50 mV/sec.
3. Thereafter, a current-potential curve at the rotation speed of 1600 rpm was measured at a scanning potential of 135 mV to 1085 mV vs. RHE and a scanning speed of 10 mV/sec.
4. The electrolyte was saturated with oxygen gas for not less than 15 minutes, and a current-potential curve at a rotation speed of 1600 rpm was measured at a scanning potential of 135 mV to 1085 mV vs. RHE and a scanning speed of 10 mV/sec.

Fig. 4 shows a current-potential curve at a rotation speed of 1600 rpm for each catalyst. The value of electric current at each electric potential in each graph in Fig. 4 is obtained by subtracting the current value obtained in the evaluation condition 3 from the current value obtained in the evaluation condition 4. The current value obtained in the evaluation condition 4 includes an electric current for reducing the oxides on the surface of the metal catalyst particles, namely, palladium-gold alloy particles or palladium particles, in addition to an oxygen reduction current. Thus, in order to evaluate a net oxygen reduction current, it is necessary to exclude the electric current for reducing the oxides on the surface of the metal catalyst particles. As such measure, the current value obtained in the evaluation condition 3 was subtracted from the current value obtained in the evaluation condition 4 to give a net oxygen reduction current, as mentioned above.
As an index of oxygen reducibility, an oxygen reduction current at the potential of 0.85 V vs. RHE was used. A value of oxygen reduction current of each catalyst is shown in Table 6. Fig. 5 is a graphic illustration of the results shown in Table 6, wherein the graph shows a relation between the alloying degree and oxygen reduction current. A greater absolute value of the oxygen reduction current is preferred.
[Table 6]

| Sample | A | B | C | D | E (*) |
|---|---|---|---|---|---|
| I/mA · $\mu g^{-1}$ | -0.1 | -0.089 | -0.064 | -0.055 | -0.079 |
| Alloying degree (%) | 96.02 | 85.41 | 64.18 | 12.85 | - |
| (*): palladium-supported carbon catalyst | | | | | |

As shown in Table 6, a palladium-gold alloy-supported carbon catalyst having an alloying degree of as high as 96.02% or 85.41 % (sample A or B) exhibits a higher oxygen reduction potential compared with a palladium-supported carbon catalyst and is excellent in oxygen reducibility (Fig. 5). On the other hand, a palladium-gold alloy-supported carbon catalyst having an alloying degree of as low as 64.18% or 12.85% (sample C or D) exhibits a lower oxygen reduction potential compared with a palladium-supported carbon catalyst and is inferior in oxygen reducibility.
It is considered that, in a palladium-gold alloy-supported carbon catalyst having a low alloying degree, the unalloyed gold particles which have low oxygen reducibility exist near the palladium particles, which suggests the possibility that gold particles hinder the oxygen-reduction reaction conducted by palladium. On the other hand, it is considered that, in the palladium-gold alloy-supported carbon catalyst having a high alloying degree, the electronic state of the palladium is changed by the alloying, whereby a superior oxygen reducibility is exhibited compared with the case where palladium is used alone.

(4) Selective hydrogenation reaction of aliphatic nitrile compound

A hydrogenation reaction of valeronitrile was carried out under the conditions shown in Table 7.
[Table 7]

| | |
|---|---|
| Substrate | valeronitrile 3.4g (molecular weight: 83.13) 41mmol |
| Catalyst | An amount which provides a content of metals in the catalyst of 0.44 mol % relative to the substrate |
| Solvent | 50 mL |
| Atmosphere | $H_2$ (0.15 MPa = 1.48 atm) |
| Temperature | 50°C |
| Time | 3 hours |

Valeronitrile (3.4 g) was used as a model substrate. The substrate and the solvent 50 mL were introduced into a reaction vessel to give a suspension. Then, a catalyst was introduced thereto in an amount corresponding to 0.44 mol % when reduced to the content of the metals in the catalyst relative to the substrate, and the resulting mixture was stirred in the hydrogen atmosphere (0.15 MPa=1.48 atm) at 50 °C. Three hours after, the resulting reaction liquid was partitioned between pure water and hexane and extracted. (when acetic acid was used as a solvent, the partition/extraction was effected after preliminarily neutralization with a caustic soda solution). Finally, an appropriate amount of nitrobenzene that is an inner standard substance was added to the upper layer (hexane layer) for sampling. The resulting sample was analyzed by gas chromatography (GC). Incidentally, the hydrogenation reaction of valeronitrile proceeds according to the following reaction scheme and generates amines 1 to 3 shown in the scheme.

[Chemical formula 2]

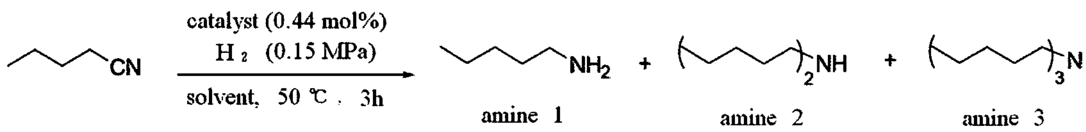


In Table 8 are shown the combinations of catalyst and solvent that were used, as well as the results of the measurements of conversion ratio and selectivity. In the table, the conversion ratio is a weight ratio of the weight of consumed substrate to the weight of the substrate before the reaction, and the selectivity is shown by a mol ratio of amines 1,2 and 3.

[Table 8]

| Experiment | Catalyst | solvent(*) | Conversion ratio (%) | Selectivity (amine 1:amine 2: amine 3) |
|---|---|---|---|---|
| 1 | 10wt%Pd/carbon | MeOH | 24.4 | 0 : 11 : 68 |
| 2 | 10wt%Pd/carbon | THF | 63.8 | 0 : 4 : 89 |
| 3 | 10wt%Pd/carbon | AcOH | 100 | 56 : 29 : 7 |
| 4 | 30wt%Pd/carbon | AcOH | 100 | 51 : 36 : 1 |
| 5 | 30wt%Au/carbon | AcOH | 0 | No reaction |
| 6 | Mixture of 30wt%Pd/carbon and 30wt%Au/carbon (supported metal ratio: Pd 25wt%, Au 5.2wt%) | AcOH | 100 | 30 : 25 : 7 |
| 7 | 25wt% Pd - 5.2wt% Au/carbon (Sample A, alloying degree: 96.02%) | AcOH | 100 | 72 : 26 : 2 |
| 8 | 25wt% Pd - 5.2wt% Au/carbon (Sample D, alloying degree: 12.85%) | AcOH | 100 | 29 : 34 : 11 |

(*) MeOH: methanol, THF: tetrahydrofuran, AcOH: acetic acid

At first, a 10 wt% Pd/carbon was used as a catalyst, and MeOH, THF or AcOH was used as a reaction solvent to carry out a hydrogenation reaction. As the results, it was revealed that a raw material remained and an aimed primary amine was not produced at all (Experiments 1 and 2). On the other hand, a dramatic enhancement in conversion ratio and selectivity was observed in the reaction in AcOH (Experiment 3).
It is considered that the result comes from the suppression of the addition of a primary amine to a synthetic intermediate imine, as previously explained.

Then a hydrogenation reaction was carried out in a solvent ofAcOH using a 30 wt% Pd/carbon, which resulted in an activity equivalent to that in Experiment 3 (Experiment 4). On the other hand, hydrogenation of the nitrile did not proceed at all with a 30 wt% Au/carbon catalyst (Experiment 5). From this result, it was revealed that gold does not have

a reduction activity on cyano groups at all.

Then, a measurement was conducted using a 25wt% Pd - 5.2wt% Au/carbon catalyst having an alloying degree of as high as 96.02% (the Sample A). It was found that the selectivity of a primary amine increased to 72% (Experiment 7). In order to confirm the effect of this alloy, a measurement was conducted similarly using a 25wt% Pd - 5.2wt% Au/carbon catalyst having an alloying degree of as low as 12.85% (the Sample D). It was revealed that the selectivity decreased largely to 29% (Experiment 8). It is considered that the result comes from blocking of the activity points of palladium by inactive gold particles that are not alloyed. A similar measurement was conducted by mixing 30wt%Pd/carbon and 30wt%Au/carbon in such amounts to give supported amounts of 25wt% Pd and 5.2wt% Au relative to the weight of the catalyst, and the results obtained were similar to those obtained by using the 25wt% Pd - 5.2wt% Au/carbon catalyst having a low alloying degree (Experiments 6 and 8). From these observations, it is considered that a mere mixture of palladium and gold provides a low activity whereas alloying of palladium and gold provides an enhanced selectivity of primary amine synthesis as a result of change of the electronic state of palladium.

## Claims

1. A reduction catalyst which comprises conductive carbon and palladium-gold alloy supported on the carbon, wherein the alloying degree of said alloy is in a range of from 50 to 100%.

2. The catalyst according to claim 1, wherein the catalyst is an oxygen-reduction catalyst.

3. The catalyst according to claim 2, wherein the catalyst is a cathode catalyst for a polymer electrolyte fuel cell.

4. The catalyst according to claim 1, wherein the catalyst is a hydrogenation catalyst.

5. The catalyst according to claim 4, wherein the hydrogenation is a reaction to convert an aliphatic nitrile compound to a primary amine by adding hydrogen atoms to the aliphatic nitrile compound.

6. The catalyst according to claim 1, wherein the amount of the alloy is 10 to 40% by weight relative to the weight of the catalyst.

7. A cathode for a polymer electrolyte fuel cell in which the catalyst according to claim 3 is used.

8. A method for reducing oxygen which comprises contacting the reduction catalyst according to claim 1 with oxygen.

9. A method for hydrogenating an organic compound having reducible functional groups, which comprises contacting the reduction catalyst according to claim 1 with said organic compound.

[Fig 1]

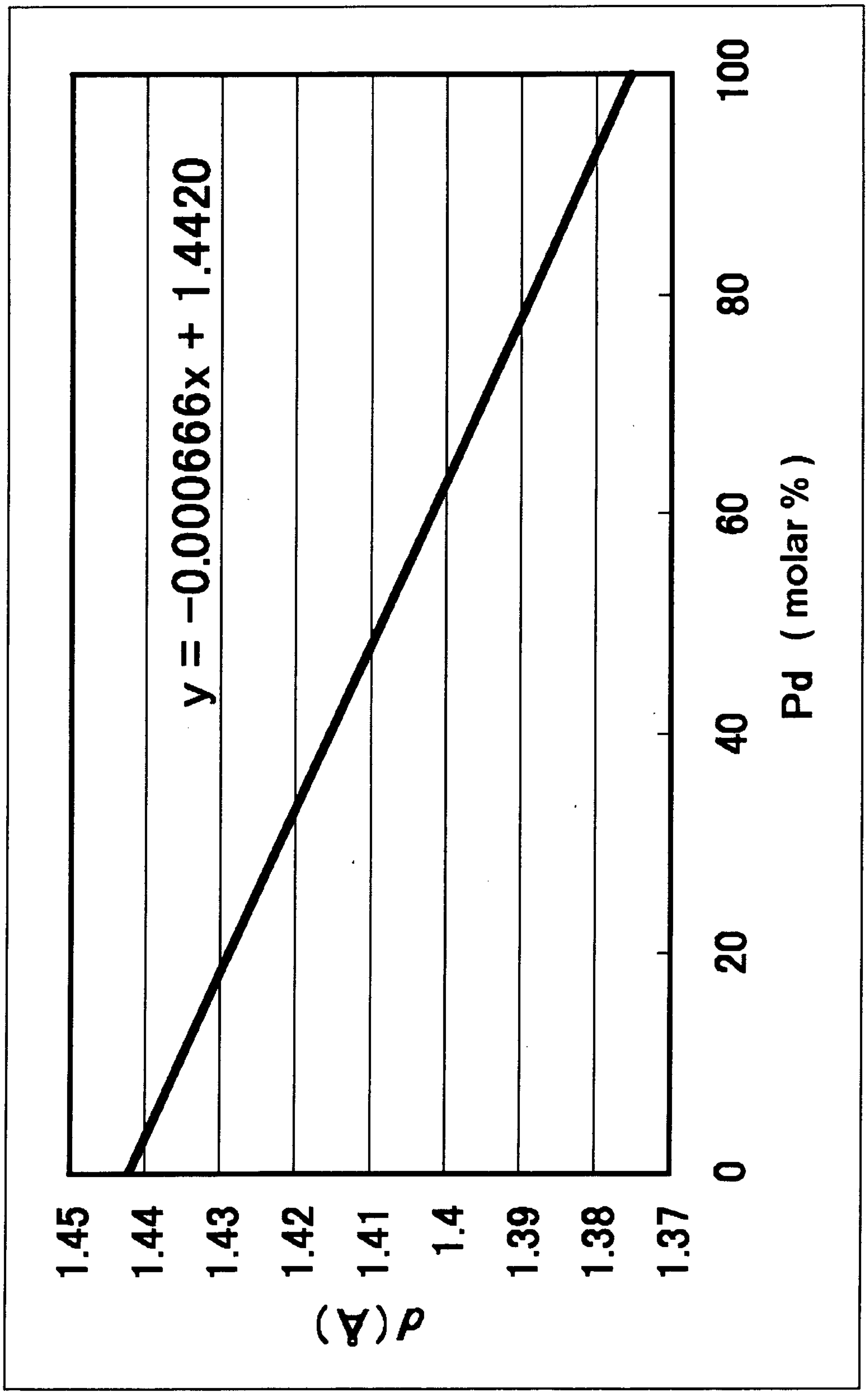
y = -0.000666x + 1.4420
d (Å)
1.45
1.44
1.43
1.42
1.41
1.4
1.39
1.38
1.37
0
20
40
60
80
100
Pd (molar %)

[Fig 2]

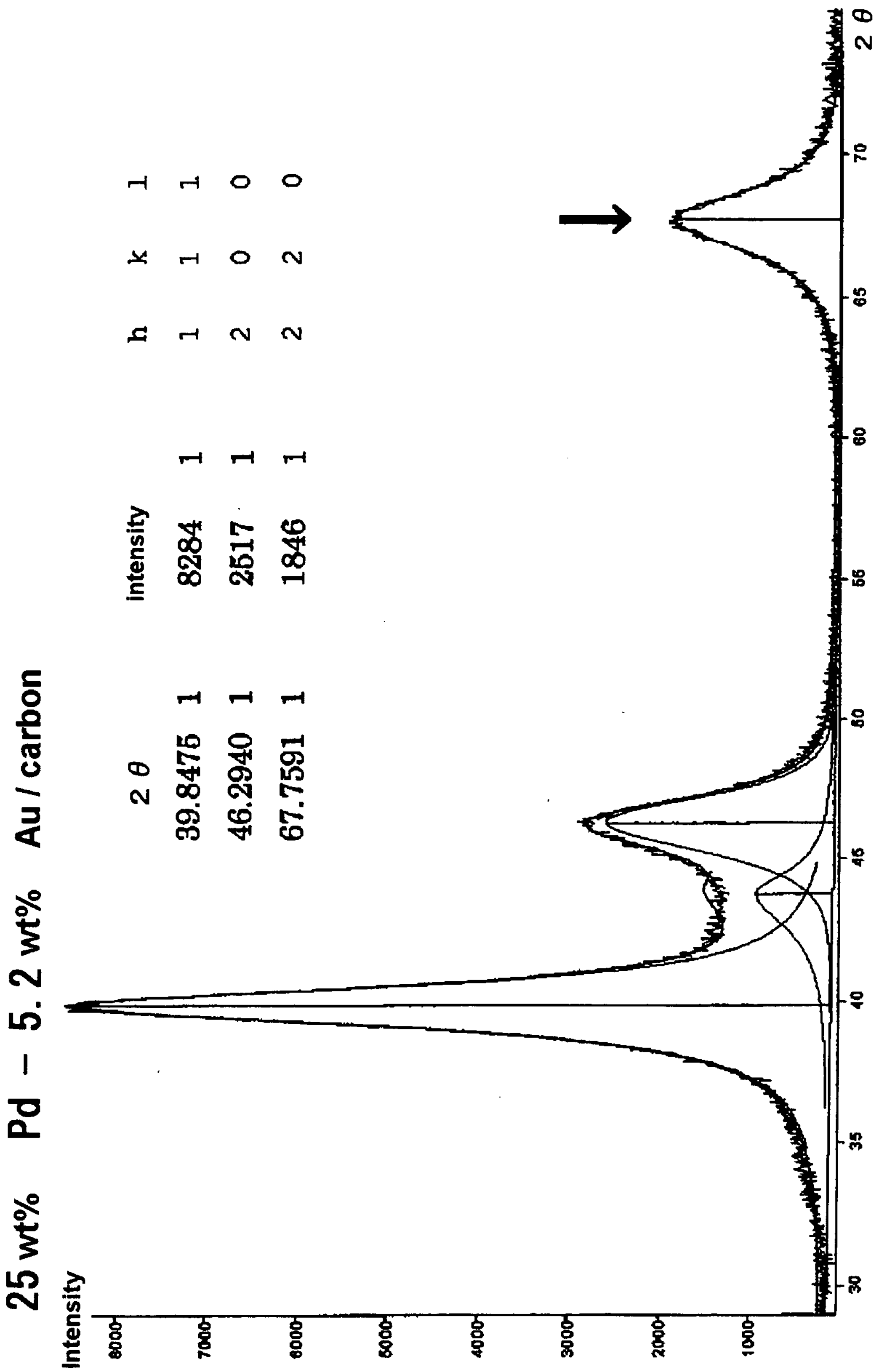
25 wt% Pd − 5. 2 wt% Au / carbon
Intensity
2 θ intensity h k l
39.8475 1 8284 1 1 1 1
46.2940 1 2517 1 2 0 0
67.7591 1 1846 1 2 2 0
8000
7000
6000
5000
4000
3000
2000
1000
30
35
40
45
50
55
60
65
70
2 θ

[Fig 3]

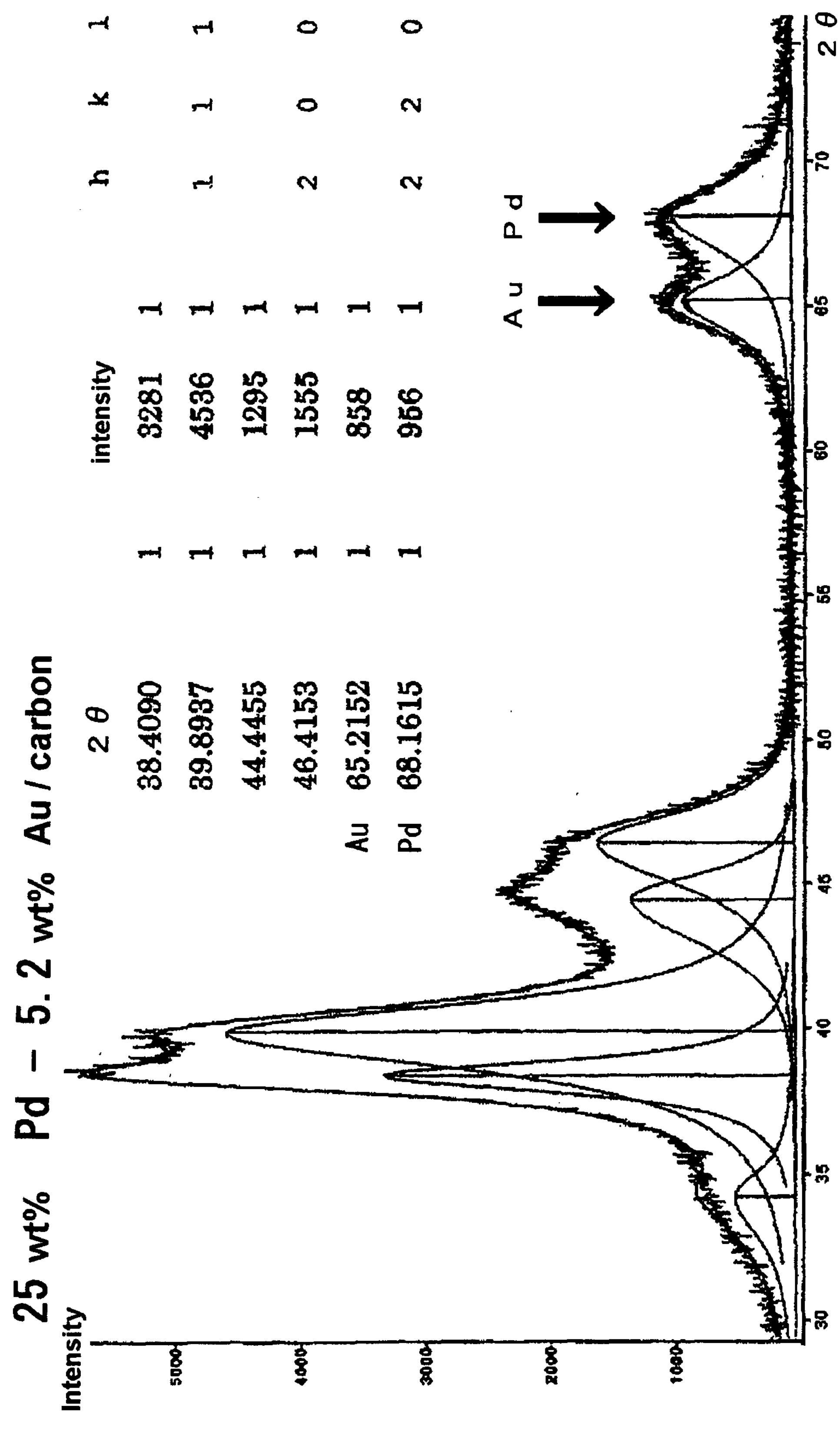
25 wt% Pd – 5. 2 wt% Au / carbon
Intensity
5000
4000
3000
2000
1000
30
35
40
45
50
55
60
65
70
2θ
2θ intensity h k l
38.4090 1 3281 1
39.8937 1 4536 1 1 1 1
44.4455 1 1295 1
46.4153 1 1555 1 2 0 0
Au 65.2152 1 858 1
Pd 68.1615 1 956 1 2 2 0
Au
Pd

[Fig 4]

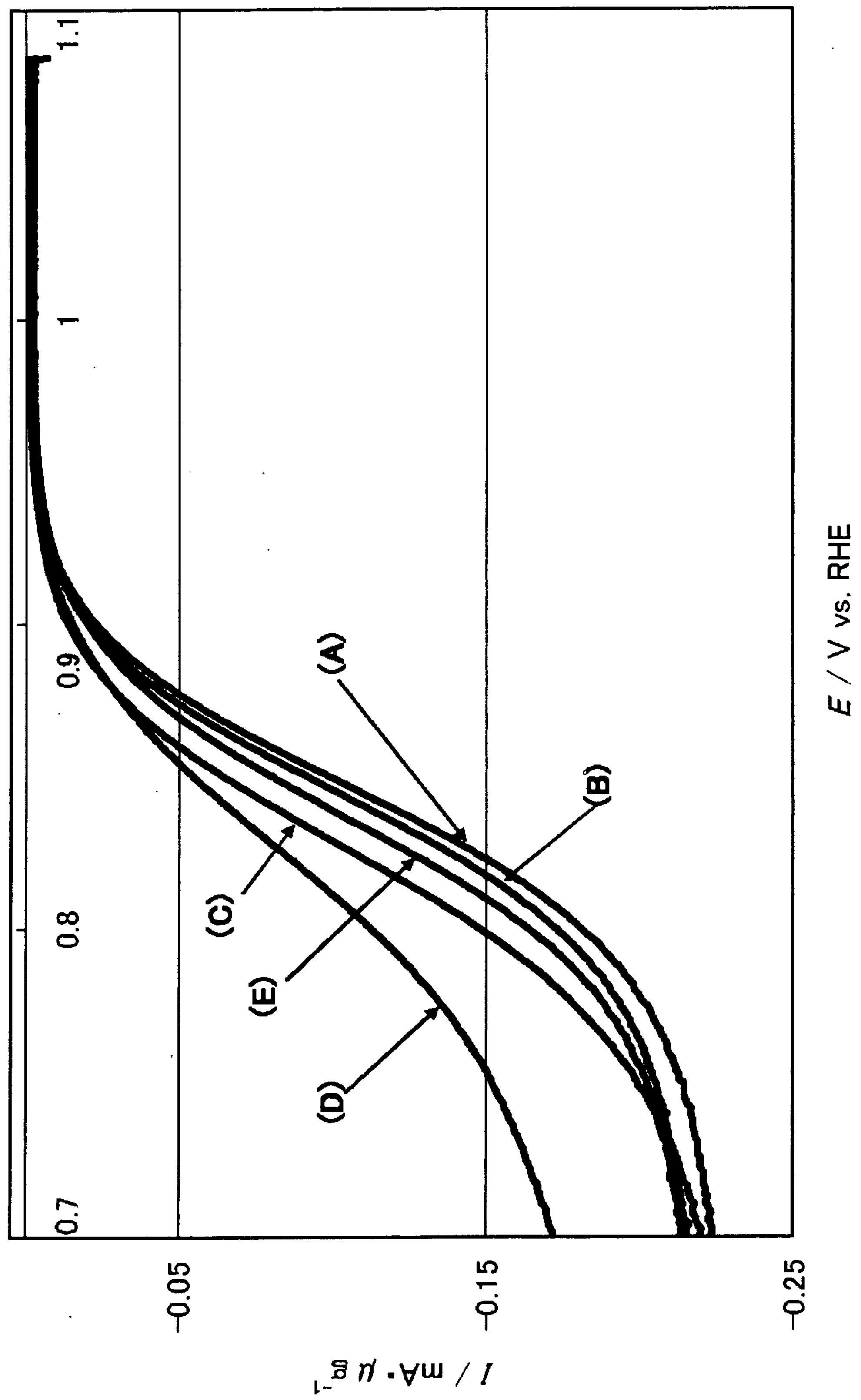

[Fig 5]

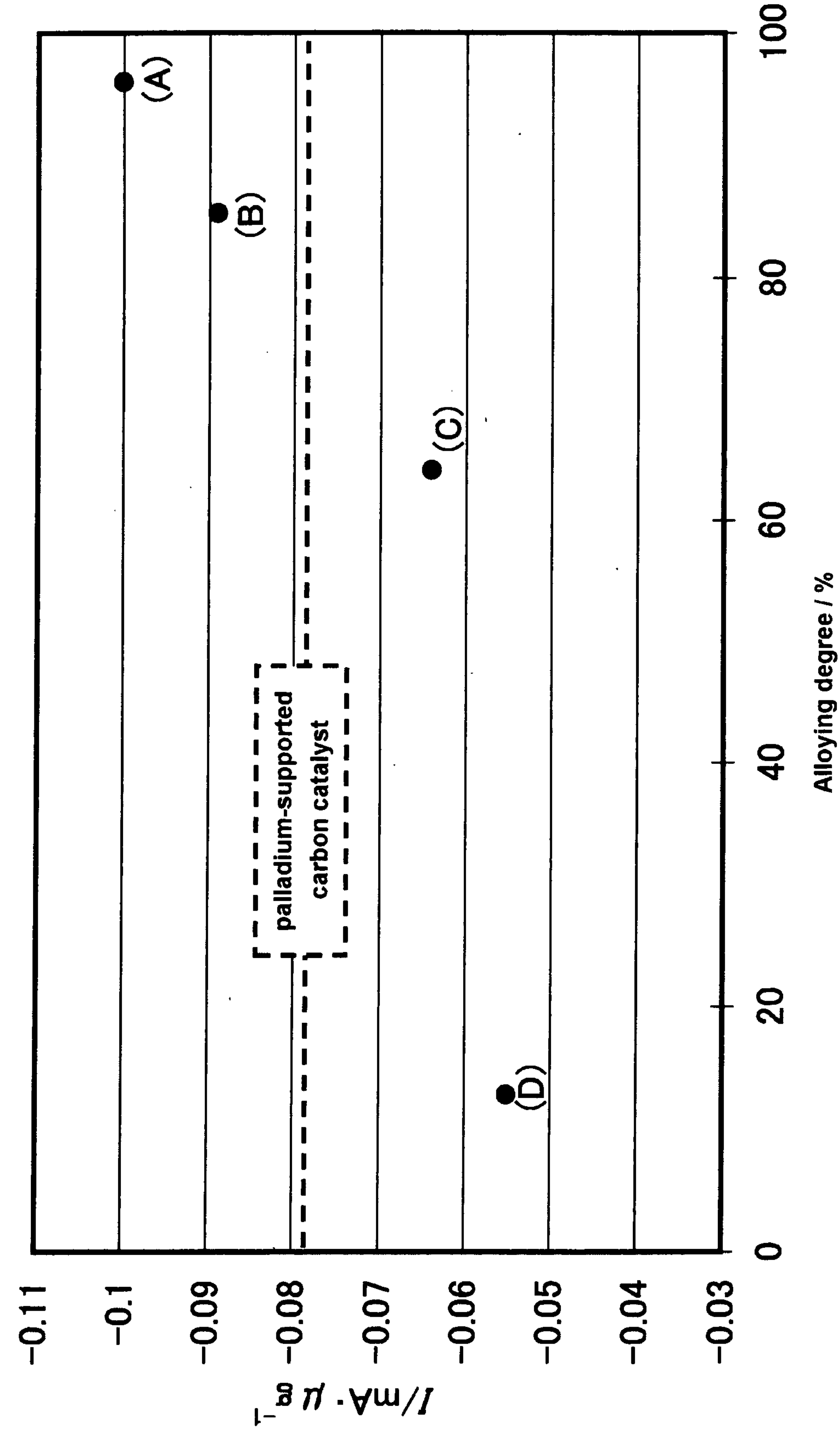

(A)
(B)
(C)
(D)
palladium-supported carbon catalyst
-0.11
-0.1
-0.09
-0.08
-0.07
-0.06
-0.05
-0.04
-0.03
0
20
40
60
80
100
Alloying degree / %
I/mA· μg-1

**INTERNATIONAL SEARCH REPORT**

International application No.
PCT/JP2011/074715

A. CLASSIFICATION OF SUBJECT MATTER
*B01J23/52*(2006.01)i, *H01M4/90*(2006.01)i, *C07B61/00*(2006.01)n, *C07C209/48*(2006.01)n, *C07C211/07*(2006.01)n

According to International Patent Classification (IPC) or to both national classification and IPC

B. FIELDS SEARCHED

Minimum documentation searched (classification system followed by classification symbols)
B01J23/52, H01M4/90, C07B61/00, C07C209/48, C07C211/07

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JST7580(JDreamII), WPI

C. DOCUMENTS CONSIDERED TO BE RELEVANT

| Category* | Citation of document, with indication, where appropriate, of the relevant passages | Relevant to claim No. |
|---|---|---|
| X<br>Y | XU, J.B. et al, Synthesis and characterization of the Au-modified Pd cathode catalyst for alkaline direct ethanol fuel cells, Int. J. Hydrogen Energy, 2010.08.03, Vol.35, No.18, p.9693-9700, doi:10.1016/j.ijhydene.2010.06.074 | 1-3,6-8<br>4,9 |
| X<br>Y | NIE, M. et al, Nanocrystaline tungsten carbide supported Au-Pd electrocatalyst for oxygen reduction, Journal of Power Sources, 2007.01.12, Vol.167, No.1, p.69-73, doi:10.1016/j.jpowsour.2006.12.059 | 1-3,6-8<br>1-4,7-9 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 January, 2012 (18.01.12) | 31 January, 2012 (31.01.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No. PCT/JP2011/074715

C (Continuation). DOCUMENTS CONSIDERED TO BE RELEVANT

| Category* | Citation of document, with indication, where appropriate, of the relevant passages | Relevant to claim No. |
|---|---|---|
| Y | KOBAYASHI, H. et al, Atomic-level Pd-Au alloying and controllable hydrogen-absorption properties in size-controlled nanoparticles synthesized by hydrogen reduction, Chemical Communications, 2009.07.02, No.32, p.4806-4808, DOI:10.1039/B907875D | 1-3,7,8 |
| Y | KOBAYASHI, H. et al, Electronic Supplementary Information for Atomic-Level Pd-Au Alloying and Hydrogen-Absorption Properties in Nanoparticles Synthesized by Hydrogen Reduction, Chemical Communications, 2009.07.02, No.32, p.1-5 | 1-3,7,8 |
| Y | YANAGISAWA, T. et al, Thermal properties of alloyed noble metal colloids in low-middle temperature region, TECHNICAL REPORT 2004, 2003.12.15, p.10-12, http://www.socnb.com/report/ptech_e/2004p10_e.pdf | 1-3,7,8 |
| Y | JP 11-506044 A (E.I. Du Pont De Nemours & Co.), 02 June 1999 (02.06.1999), examples & US 5629462 A & US 5447896 A & EP 828557 A1 & WO 1996/038225 A1 | 4,9 |
| Y | BONAROWSKA, M. et al, Hydrodechlorination of CCl2F2 (CFC-12) over Pd-Au/C catalysts, Applied Catalysis B: Environmental, 2001.11.27, Vol.35, No.1, p.13-20, doi:10.1016/S0926-3373(01)00227-2 | 4,9 |
| A | JP 2007-313423 A (Sumitomo Electric Industries, Ltd.), 06 December 2007 (06.12.2007), entire text (Family: none) | 1-3,6-8 |
| A | JP 2009-510705 A (Brookhaven Science Associates), 12 March 2009 (12.03.2009), entire text & US 2007/0026292 A1 & EP 1922777 A2 & WO 2008/033113 A2 | 1-3,6-8 |
| A | JP 9-33455 A (Sumitomo Metal Industries, Ltd.), 07 February 1997 (07.02.1997), entire text (Family: none) | 1-3,6-8 |

Form PCT/ISA/210 (continuation of second sheet) (July 2009)

**INTERNATIONAL SEARCH REPORT**

International application No.
PCT/JP2011/074715

C (Continuation). DOCUMENTS CONSIDERED TO BE RELEVANT

| Category* | Citation of document, with indication, where appropriate, of the relevant passages | Relevant to claim No. |
|---|---|---|
| A | JP 2001-514962 A (Celanese Chemicals Europe GmbH), 18 September 2001 (18.09.2001), paragraphs [0029], [0030], [0055], [0062] to [0065], [0082] to [0084], [0122] to [0126]; examples & US 6603038 B1 & US 2003/0187294 A1 & EP 1015108 A1 & WO 1999/008791 A1 | 4,9 |

Form PCT/ISA/210 (continuation of second sheet) (July 2009)

**INTERNATIONAL SEARCH REPORT**

International application No.
PCT/JP2011/074715

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The invention of claim 1 does not have novelty and a special technical feature since the invention is disclosed in the documents 1 and 2, and consequently, the invention of claim 1 does not comply with the requirement of unity.

The inventions of claims indicated below are relevant to a main invention group.

Claims 1-3, 6-8

(continued to extra sheet)

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2011/074715

Continuation of Box No.III of continuation of first sheet(2)

Document 1: XU, J.B. et al, Synthesis and characterization of the Au-modified Pd cathode catalyst for alkaline direct ethanol fuel cells, Int. J. Hydrogen Energy, 2010.08.03, Vol.35, No.18, p.9693-9700,

Document 2: NIE, M. et al, Nanocrystaline tungsten carbide supported Au-Pd electrocatalyst for oxygen reduction, Journal of Power Sources, 2007.01.12, Vol.167, No.1, p.69-73,

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SHIGEO NISHIMURA ; YUZURU TAKAGI.** Catalytic Hydrogenation Reaction, Application in Organic Synthesis. Tokyo Kagaku Dojin Co. Ltd, 10 April 1987, 34-37